Europäisches Patentamt

European Patent Office

Office européen des brevets

(11) Publication number: **0 202 112**
**A1**

(12) **EUROPEAN PATENT APPLICATION**

(21) Application number: **86303634.9**

(22) Date of filing: **13.05.86**

(51) Int. Cl.⁴: **A 61 K 31/557**
**A 61 K 9/14**
**//C07C177/00**

(30) Priority: **15.05.85 GB 8512344**

(43) Date of publication of application:
**20.11.86 Bulletin 86/47**

(84) Designated Contracting States:
**AT BE CH DE FR GB IT LI LU NL SE**

(71) Applicant: **MAY & BAKER LIMITED**

**Dagenham Essex RM10 7XS(GB)**

(72) Inventor: **Bell, John Howard**
**May & Baker Limited**
**Dagenham Essex, RM10 7XS(GB)**

(72) Inventor: **Clay, Robert Timothy**
**May & Baker Limited**
**Dagenham Essex, RM10 7XS(GB)**

(72) Inventor: **Cook, Robert Stanley**
**May & Baker Limited**
**Dagenham Essex, RM10 7XS(GB)**

(74) Representative: **Bentham, Stephen et al,**
**J.A. Kemp & Co. 14 South Square Gray's Inn**
**London WC1R 5EU(GB)**

(54) New pharmaceutical compositions.

㉗ The invention provides pharmaceutical compositions which comprise particles of a water-soluble pharmaceutically acceptable carrier, which carrier consists essentially of a neutral inorganic salt, a hexahydric alcohol or a neutral mono- or di-saccharide, and which particles have on their surface a substantially uniform film of oxoprostol, the particle size of the carrier being such that on mixing with water, rapid dissolution of the carrier takes place, and a process for their preparation.

EP 0 202 112 A1

DESCRIPTION

"NEW PHARMACEUTICAL COMPOSITIONS"

This invention relates to pharmaceutical compositions comprising a prostanol derivative as active ingredient.

The known prostanol derivative $(\pm)$-2-hydroxymethyl-16-phenoxy-15-oxo-13,14-dihydro-2-decarboxy-11,15-dideoxy-17,18,19,20-tetranorprostaglandin E, (described, for example, in British Patent No. 1581886 and hereinafter referred to as 'oxoprostol') has particular utility in the alleviation of gastric ulceration and gastric hyperacidity, more especially by oral administration. Oxoprostol is an oil of very low solubility in water or aqueous media.

According to the present invention, there are provided pharmaceutical compositions comprising particles of a water-soluble pharmaceutically acceptable carrier, which carrier consists essentially of particles of a neutral inorganic salt, for example sodium chloride, or a hexahydric alcohol, for example sorbitol or mannitol, or a neutral mono- or di-saccharide, in particular lactose, and which particles have on their surface a substantially uniform film of oxoprostol, the particle size of the carrier being such that on mixing with water rapid dissolution of the carrier takes place. (By "rapid dissolution", it is meant that when 0.5 g of the carrier is added to 500 ml of water at 37°C in a standard U.S.P. paddle dissolution apparatus, with a stirring rate of 50 revolutions per minute, no particles of the carrier remain visible to the naked eye after 2.5 minutes.) The more rapidly soluble the carrier is in water, the larger its particle size may be but, in general, carriers comprising greater than 95% by weight of particles having a maximum dimension of from 1 to 400 microns are suitable. Preferably the particles have a median maximum dimension of not greater than 200 microns. Lactose, anhydrous, U.S.P. is preferred as the carrier. Preferably, the carrier contains not more than 1.5% w/w (weight/weight), and more particularly not more than 1% w/w, of water.

The ratio by weight of oxoprostol to carrier may be from 0.0001:1 to 1:1 and is preferably from 0.005:1 to 0.1:1.

The particulate pharmaceutical compositions according to the present invention possess the particularly advantageous and unexpected property that when mixed with water or a substantially aqueous media, for example the contents of the stomach, a relatively stable emulsion is formed, without the necessity of incorporating surface-active agents, which renders them particularly suitable for oral administration for the alleviation of gastric ulceration and gastric hyperacidity by making the oxoprostol available for local action and producing good absorption through the gastric lining.   Thus, a particulate pharmaceutical composition according to the present invention comprising oxoprostol coated on the surface of particles of Lactose, anhydrous, U.S.P. in the ratio by weight of oxoprostol to Lactose, anhydrous, U.S.P. of 0.02 to 1, when dissolved with gentle agitation in water in the amount of 1 g of composition to 10 ml of water, forms an emulsion which is stable under visual examination for two days.

Particulate pharmaceutical compositions according to the present invention may be prepared by dissolving the oxoprostol in an inert organic solvent or a mixture of inert organic solvents in which the oxoprostol is soluble, and preferably at least 5% weight/volume (w/v) soluble, and in which the carrier is substantially insoluble, i.e. preferably not more than 0.1% w/v soluble, contacting the oxoprostol solution with the carrier, for example by spraying the solution onto the carrier or mixing the solution with the carrier with agitation, to achieve uniform coating of the carrier particles with the oxoprostol solution, and drying the product to remove the organic solvent. The temperature at which the product is dried will depend on the solvent selected for the coating process but should not exceed 80°C and should preferably be below 45°C.   Reduced atmospheric pressure may
advantageously be used to increase the rate of solvent
evaporation at any given temperature.   Suitable
solvents have boiling points lower than about 100°C,
and preferably lower than 50°C, measured at normal
atmospheric pressure.   Dichloromethane is a
particularly suitable solvent.

The presence of impurities, and in particular
basic impurities, in the solvent may cause catalytic
degeneration of the oxoprostol and, accordingly, the
use of solvents containing such impurities should be
avoided.

For oral administration for the alleviation of
gastric ulceration and gastric hyperacidity,
particulate pharmaceutical compositions according to
the present invention may be dissolved in water and the
emulsion thus obtained may be administered orally or,
as is preferred, particulate pharmaceutical
compositions according to the present invention may be
formulated into conventional pharmaceutical
compositions suitable for oral administration, for
example compressed into tablets or placed in capsules
of absorbable material such as gelatin, with
conventional excipients, for example diluents, e.g.
lactose, disintegrating agents, e.g. sodium starch
glycollate or microcrystalline cellulose, and small
amounts, e.g. up to 5% w/w, of lubricating agents or
glidants, e.g. stearic acid, magnesium stearate, sodium

lauryl sulphate, magnesium lauryl sulphate or talc, to facilitate the handling and flow properties of the composition.

Particulate pharmaceutical compositions according to the invention may, if desired, be prepared and stored under sterile, aseptic conditions and mixed with sterile water at the time of use, to give extemporaneous sterile emulsions containing oxoprostol suitable for parenteral administration, e.g. intravenous injection.

For the treatment of adult patients with gastric ulceration and of adult patients with gastric hyperacidity, pharmaceutical compositions according to the present invention may normally be administered at rates which give oral doses of between 0.001 and 1.0 mg, and particularly of between 0.001 and 0.02 mg, of oxoprostol per kg body weight administered 2, 3 or 4 times per day, or doses of between 0.002 and 2.0 mg of oxoprostol per kg body weight by intravenous injection.

The following non-limitative Examples illustrate the present invention:-

EXAMPLE 1

A solution of oxoprostol (0.52 g) in dichloromethane (20 ml) was added in small portions to Lactose, anhydrous, U.S.P. (10.4 g) over a period of 30 minutes whilst the lactose was continuously stirred in a mixer. The dichloromethane was then allowed to evaporate at ambient temperature over a period of 30 minutes.

The coated lactose particles thus obtained (10.0 g) were placed in a sealable glass jar to which Lactose, anhydrous, U.S.P. (89.5 g) was then added. The jar was placed on a Turbula TC2 mixer and mixed for thirty minutes. Magnesium stearate (0.5 g) was then added and mixing was continued for 1 minute.

A sample (1 g) of the powder thus obtained was added to water (10 ml) with gentle agitation. An emulsion in which no individual oily droplets could be distinguished by the naked eye and which remained stable and visually unchanged for two days was thus obtained. Similar results were obtained when the water was replaced by 0.01N and 0.1N hydrochloric acid.

The remainder of the powder was then filled in 100 g portions (each containing 0.5 mg of oxoprostol) into No. 3 hard gelatin capsules using a Tevopharm semi-automatic capsule filling machine.

A capsule thus obtained was placed in a standard dissolution vessel containing 0.1N hydrochloric acid (600 ml) and formed in 3 to 4 minutes a dilute emulsion in which no individual oily droplets could be distinguished by the naked eye.

## EXAMPLE 2

Oxoprostol (0.52 g) was dissolved in dichloromethane (20 ml) and the solution thus obtained was sterilized by passage through a 0.2 micron solvent resistant filter. Lactose, anhydrous, U.S.P. (10.4 g) was sterilized by dry heating in an oven at 150°C for one hour. The solution was then added to the lactose over a period of 30 minutes with continuous stirring in a mixer under aseptic conditions. The dichloromethane was then allowed to evaporate over a period of 30 minutes at ambient temperature, again under aseptic conditions. The powder was then filled in 1 g portions into sterile glass vials (20 mls), which were then sealed with a rubber plug. The introduction of sterile water for injection (10 ml) through the rubber plug by means of a hypodermic needle, gave a sterile emulsion suitable for injection, which could be withdrawn through the plug by means of the hypodermic needle, each ml of emulsion containing 0.5 mg of oxoprostol.

## CLAIMS

1. A pharmaceutical composition which comprises particles of a water-soluble pharmaceutically acceptable carrier, which carrier consists essentially of a neutral inorganic salt, a hexahydric alcohol or a neutral mono- or di-saccharide, and which particles have on their surface a substantially uniform film of oxoprostol, the particle size of the carrier being such that on mixing with water, rapid dissolution of the carrier takes place.

2. A composition according to claim 1 in which the carrier is sodium chloride, sorbitol, mannitol or lactose.

3. A composition according to claim 1 in which the carrier is lactose.

4. A composition according to claim 1, 2 or 3 in which the carrier comprises more than 95% by weight of particles having a maximum dimension of from 1 to 400 microns.

5. A composition according to any one of the preceding claims in which the carrier comprises particles having a median maximum dimension not greater than 200 microns.

6. A composition according to any one of the preceding claims in which the carrier contains not more than 1.5% by weight of water.

7. A composition according to any one of the preceding claims in which the carrier contains not more than 1% by weight of water.

8. A composition according to any one of the preceding claims in which the carrier is anhydrous lactose.

9. A composition according to any one of the preceding claims in which the ratio by weight of oxoprostol to carrier is from 0.0001:1 to 1:1 and preferably from 0.005:1 to 0.01:1.

10. A process for the preparation of a pharmaceutical composition according to claim 1 which comprises dissolving oxoprostol in an inert organic solvent or a mixture of inert organic solvents in which the oxoprostol is soluble, preferably at least 5% weight/volume soluble, and in which the carrier is substantially insoluble, preferably not more than 0.1% weight/volume soluble, contacting the oxoprostol solution with the carrier to achieve uniform coating of the carrier particles with the oxoprostol solution and drying the product to remove organic solvent, the preferred organic solvent being dichloromethane.

0202112

## European Patent Office

## EUROPEAN SEARCH REPORT

Application number

| | DOCUMENTS CONSIDERED TO BE RELEVANT | | EP 86303634.9 |
|---|---|---|---|

| Category | Citation of document with indication where appropriate, of relevant passages | Relevant to claim | CLASSIFICATION OF THE APPLICATION (Int Cl.4) |
|---|---|---|---|
| Y | DE - A1 - 2 822 751 (MAY & BAKER LTD.)<br>* Claim 7; page 6, lines 1-29; page 13, lines 11-17 * | 1-3,10 | A 61 K 31/557<br>A 61 K 9/14<br>/C 07 C 177/00 |
| Y | GB - A - 1 578 998 (G.D.SEARLE & CO.)<br>* Example 6, lines 20-29, 36-43 * | 1-3,10 | |
| A | DE - A1 - 2 751 920 (G.D.SEARLE &CO)<br>* Page 17, lines 6-18; page 18, lines 16-23 * | 1-3,10 | |
| | | | **TECHNICAL FIELDS SEARCHED (Int. Cl.4)**<br><br>A 61 K 31/00<br>A 61 K 9/00<br>C 07 C 177/00 |

The present search report has been drawn up for all claims

| Place of search<br>VIENNA | Date of completion of the search<br>19-08-1986 | Examiner<br>MAZZUCCO |
|---|---|---|